# EUROPÄISCHE PATENTSCHRIFT

(11) **EP 3 735 205 B1**
(45) Veröffentlichungstag und Bekanntmachung des Hinweises auf die Patenterteilung: **29.01.2025**
(21) Anmeldenummer: 18829332.8
(22) Anmeldetag: 18.12.2018
(51) Int. Cl.: A61F 2/58, A61F 2/68

(54) **GREIFEINRICHTUNG**
GRIPPING DEVICE
DISPOSITIF DE PRÉHENSION

(30) Priorität: 05.01.2018 DE 102018100173
(43) Veröffentlichungstag der Anmeldung: 11.11.2020
(73) Patentinhaber: Otto Bock Healthcare Products GmbH, 1110 Wien (AT)
(72) Erfinder: EDER, Florian, 1040 Wien (AT)
(74) Vertreter: Gramm, Lins & Partner Patent- und Rechtsanwälte PartGmbB
(86) Internationale Anmeldenummer: PCT/EP2018/085659
(87) Internationale Veröffentlichungsnummer: WO 2019/134826

(56) Entgegenhaltungen:
- WO-A2-2007/076763
- DE-A1- 102014 007 743
- US-A- 1 507 681
- US-A- 3 694 021

## Beschreibung

Die Erfindung betrifft eine Greifeinrichtung mit einem Chassis, zumindest einem an dem Chassis schwenkbar gelagerten ersten Fingerelement, zumindest einem Antrieb und einem Kraftübertragungselement, das den Antrieb mit dem ersten Fingerelement koppelt. Die Greifeinrichtung ist als orthopädietechnische Greifeinrichtung ausgebildet und zur Anwendung als Prothesenhand oder Teil einer Prothesenhand geeignet.

Die Aufgabe einer Greifeinrichtung ist es, Gegenstände zu greifen und in der gegriffenen Position festzuhalten. Unterschiedliche Gegenstände benötigen unterschiedliche Greifeinrichtungen, um optimal gegriffen werden zu können. Die einfachste Greifeinrichtung besteht aus zwei, um eine starre Schwenkachse zueinander verlagerbaren, stabartigen Greifelementen, die auf einander zu oder voneinander weg bewegt werden. Mit einer solchen Greifeinrichtung sind beispielsweise flache Gegenstände leicht zu greifen. Problematisch ist es, wenn eine Greifeinrichtung eine Vielzahl von Gegenständen greifen können soll oder in der Lage sein soll, eine Vielzahl von Gegenständen mit unterschiedlichen Formen zu greifen. Eine solche Aufgabenstellung stellt sich insbesondere bei der Anwendung der Greifeinrichtung als eine orthopädietechnische Greifeinrichtung in einer Handprothese, da ein Patient oder ein Nutzer einer Handprothese im täglichen Gebrauch mit einer Vielzahl unterschiedlicher Gegenstände konfrontiert ist, die gegriffen und gehalten werden sollen. Darüber hinaus besteht die Notwendigkeit bei einer Handprothese, neben der Funktion auch die Optik einer Hand zu ersetzen. Dazu sind Fingerelemente an einem Chassis gelagert.

Aus der US 2004/00195638 A1 ist ein Zwei-Finger-Greifer bekannt, bei dem zwei Greifeinrichtungen aus einer geöffneten Stellung in eine geschlossene Stellung verlagert werden können, in der die Greifeinrichtungen unmittelbar aneinander gegenüberliegen. Ein zwischen den Greifeinrichtungen befindlicher Gegenstand kann somit gehalten werden. Zum Lösen des Griffes kann eine Drehrichtungsumkehr des Antriebes eingeleitet werden.

Die WO 03/017880 A1 betrifft eine Prothesenhand, bei der jeder einzelne Prothesenfinger, der an einem Chassis gelagert ist, jeweils einen separaten Antrieb aufweist. Der Antrieb ist in dem jeweiligen Prothesenfinger angeordnet. Mit einer solchen Prothesenhand ist es möglich, verschiedene Griffsituationen zu realisieren, beispielsweise einen Spritzgriff oder einen Lateralgriff. Nachteilig daran ist der hohe Steuerungsaufwand für jeden einzelnen Finger, die aufwendige Technik mit in den Finger integrierten Antrieben sowie eine erhöhte Störanfälligkeit aufgrund der komplexen Bauweise.

Die DE 405 871 B1 beschreibt eine künstliche Hand mit einem Chassis oder Handteller, an dem drehbare Finger und ein drehbarer Daumen angeordnet sind. In dem Handteller ist eine um eine zur Handfläche senkrechte Achse drehbare Antriebscheibe durch Verbindungen mit den Fingern und dem Daumen derart angeschlossen, dass die Drehung der Scheibe in die eine Richtung das Öffnen und in entgegengesetzter Richtung das Schließen der Finger bewirkt. Die Drehung der Scheibe in die eine Richtung wird durch eine Schnur bewirkt, die Rückstellbewegung in entgegengesetzte Richtung durch eine Feder im Inneren der Scheibe. An dem Umfang der Antriebsscheibe sind Sperrzähne angebracht, die mit einer Sperrklinke in Eingriff gebracht werden und die Antriebsscheibe in derjenigen Stellung festhalten, die sie im Rahmen der Drehung durch die Schnur erreicht hat.

Die EP 1 971 297 B1 beschreibt eine Handprothese mit einem Chassis, an dem mehrere Fingerprothesen gelenkig oder elastisch gelagert sind, die über einen Antrieb um zumindest eine Schwenkachse relativ zu dem Chassis aufeinander zu bewegbar sind. Kraftübertragungseinrichtungen sind an einem gemeinsamen Antrieb über ein Kopplungselement dergestalt mit den Fingerprothesen gekoppelt, das ausgehend von einer Ruhestellung in Abhängigkeit von der Drehrichtung des Antriebs zumindest zwei Fingerprothesen unterschiedliche Verstellwinkel in dieselbe Richtung relativ zu dem Chassis durchlaufen, wobei die Kraftübertragungseinrichtungen dergestalt mit dem Kopplungselement gekoppelt sind, dass ihre antriebsseitigen Lagerungen unterschiedliche Totpunktlagen aufweisen.

Die WO 2007/076763 A2 betrifft eine Handprothese mit einem Chassis, an dem zumindest eine Fingerprothese gelenkig gelagert ist. Die Fingerprothese ist über eine Kraftübertragungseinrichtung mit dem Antrieb verbunden und um zumindest eine Schwenkachse bewegbar. Die Kraftübertragungseinrichtung zwischen dem Antrieb und der Fingerprothese ist zugstarr sowie drucknachgiebig ausgebildet.

Die DE 10 2014 007 743 A1 betrifft ein modular aufgebautes Handelement mit einem Grundelement und mehreren Fingerelementen, die aus einer Gruppe verschiedener Fingerelemente ausgebildet werden können. Ein Daumenelement ist um zwei Achsen schwenkbar an dem Grundelement gelagert. Ein Motor zum Antreiben des Daumenelementes ist innerhalb des Daumenelementes angeordnet. Seine Ausgangswelle ist mit einem Getriebe verbunden, dessen Ausgangsachse über mehrere Zahn- und/oder Schneckenräder oder sonstige Elemente mit einem Befestigungsbereich des Daumenelementes funktionell verbunden ist.

Die US 3,694,021 A beschreibt eine mechanische Hand mit einer Grundplatte, an der eine Vielzahl von Fingern beweglich angeordnet sind. Ein Daumenelement ist an der Basisplatte um drei Drehachsen gelenkig gelagert. Sowohl die Finger als auch das Daumenelement weisen eine Vielzahl von Drehgelenken auf. Die Finger und das Daumenelement werden über Zugmittel angetrieben, die eine unabhängige Bewegung der einzelnen Elemente bewirken.

Aufgabe der vorliegenden Erfindung ist es, eine Greifeinrichtung bereitzustellen, bei der auf eine vereinfachte Art und Weise unterschiedliche Griffpositionen mit einem möglichst geringen Aufwand erreicht werden können.

Erfindungsgemäß wird dies durch eine Greifeinrichtung mit den Merkmalen des Hauptanspruches gelöst. Vorteilhafte Ausgestaltungen und Weiterbildungen der Erfindungen sind in den Unteransprüchen, der Beschreibung sowie den Figuren offenbart.

Die Greifeinrichtung mit einem Chassis, zumindest einem an dem Chassis schwenkbar gelagerten ersten Fingerelement, zumindest einem Antrieb und einem Kraftübertragungselement, das den Antrieb mit dem ersten Fingerelement koppelt, sieht vor, dass das Kraftübertragungselement das erste Fingerelement um zwei unterschiedlich orientierte Schwenkachsen relativ zu dem Chassis verschwenkt. Durch die Lagerung des Fingerelementes um zwei unterschiedlich orientierte Schwenkachsen und die Verschwenkung des ersten Fingerelementes durch das Kraftübertragungselement um die beiden Schwenkachsen ist es möglich, durch nur eine Kraftübertragungseinrichtung über den Antrieb das erste Fingerelement in unterschiedliche Position zu bewegen und diese Position in einem großen Umfang festlegen zu können. Durch die Anordnung des Kraftübertragungselementes an unterschiedlichen Kopplungspunkten an dem ersten Fingerelement ist es möglich, unterschiedliche Orientierungen und Positionen durch eine Kombination der unterschiedlichen Drehbewegungen um die beiden Schwenkachsen zu erreichen, wodurch sich eine verbesserte Anpassbarkeit an den jeweiligen Nutzungszweck und eine vergrößerte Variationsbreite ergibt. Die Erfindung sieht vor, dass zumindest ein zweites Fingerelement schwenkbar an dem Chassis gelagert und mit dem Antrieb dergestalt gekoppelt ist, dass ausgehend von einer Ruhestellung in Abhängigkeit von der Drehrichtung des Antriebs das erste und zweite Fingerelement unterschiedliche Trajektorien relativ zueinander durchlaufen. Durch die unterschiedlichen Trajektorien in Abhängigkeit von dem Drehsinn des Antriebes ist es möglich, unterschiedliche Griffpositionen einzustellen, beispielsweise einen Lateralgriff und einen Spitzgriff, wobei das erste Fingerelement um mehrere Schwenkachsen drehbar ist, ohne einen weiteren Antrieb vorsehen zu müssen. Allein durch die Wahl des Drehsinnes des Antriebes kann mit einer einzigen Umschaltbewegung, beispielsweise durch elektromyografische Impulse, die Wahl des jeweils benötigten Griffes oder der Zuordnung der Fingerelemente zueinander erfolgen.

Es besteht die Möglichkeit, zusätzlich zu dem einen Antrieb, der das Fingerelement um zwei unterschiedlich orientierte Schwenkachsen relativ zu dem Chassis verschwenkt, weitere Antriebe vorzusehen, die in dem Fingerelement angeordnet sein können. So kann beispielsweise ein Fingerglied einen Antrieb aufnehmen oder aus einem Antrieb bestehen, der dann als Hilfsantrieb oder als Positionierhilfe zum Einstellen der Position des Fingergliedes dient.

Die Schwenkachsen, um die das erste Fingerelement herum verschwenkt werden kann, können senkrecht zueinander orientiert sein, wodurch sich eine einfache Auslegung der Bewegung des Fingers in Abhängigkeit von dem Antrieb erreichen lässt. Wenn sich die beiden Achsen schneiden, kann die Bewegung der einen Achse die Bewegung der anderen Achse nicht beeinflussen, bei sich nicht schneidenden Achsen kann die Verdrehung um die eine Achse ausgenutzt werden, um eine andere Bewegung einzuleiten.

Das erste Fingerelement kann an einem Träger schwenkbar um eine der Schwenkachsen gelagert sein, wobei der Träger an dem Chassis um die andere Schwenkachse schwenkbar gelagert ist. Der Träger stellt somit eine erste Verbindung zwischen dem Chassis und dem Fingerelement dar, das letztendlich mit dem zu greifenden Gegenstand in Kontakt tritt. Das Trägerelement ermöglicht die nahezu freie Positionierung der einen Schwenkachse aufgrund der Möglichkeit, den Träger nahezu beliebig zu gestalten und den Ort und die Orientierung der einen Schwenkachse frei zu wählen. Die andere Schwenkachse dient zur Befestigung des Trägers an dem Chassis, so dass diese Schwenkachse hinsichtlich der Anordnung einer gewissen Restriktion unterworfen ist. Der Träger als Zwischenstück zwischen dem Chassis und dem Fingerelement vergrößert die Designmöglichkeiten und die Orientierungen der Schwenkachsen zueinander.

Eine Variante der Erfindung sieht vor, dass das Kraftübertragungselement an dem ersten Finger befestigt ist. Die Befestigung kann eine unmittelbare Befestigung des Kraftübertragungselementes an dem Fingerelement ergeben. Ohne Zwischenschaltung weiterer Kraftübertragungselemente ist es möglich, eine direkte Kopplung zwischen dem Antrieb und dem Fingerelement zu erreichen. Das Kraftübertragungselement ist dabei nicht an dem Träger gekoppelt, sondern direkt an dem Fingerelement, so dass eine Schwenkachse, die durch den Träger an dem Chassis ausgebildet ist, nicht mit einer Befestigungsstelle des Kraftübertragungselementes an dem ersten Fingerelement zusammenfällt. Insbesondere bei einer schwenkbaren Lagerung des Kraftübertragungselementes an dem ersten Fingerelement fällt die Schwenkachse, um die das Kraftübertragungselement an dem Fingerelement gelagert ist, mit der Schwenkachse, mit der das Trägerelement an dem Chassis gelagert ist, auseinander. Dadurch können die Abstände zwischen der Lagerungsstelle des Kraftübertragungselementes an dem ersten Fingerelement und der Schwenkachse an dem Chassis verändert werden, wodurch sich die Kräfte oder Momente, die über den Antrieb übertragen werden können, verändern lassen. Ebenso können sich unterschiedliche Wegübersetzungen einstellen lassen, wodurch die Greifeinrichtung an den jeweiligen Einsatzzweck anpassbar ist.

Das Kraftübertragungselement ist in einer weiteren Ausführungsform kardanisch an dem ersten Fingerelement gelagert, wobei ein rotatorischer Freiheitsgrad um eine Schwenkachse gesperrt ist, wodurch das Fingerelement um eine der Schwenkachsen verdrehbar ist. Durch die Sperrung eines rotatorischen Freiheitsgrades ist es möglich, ein Drehmoment um eine der Schwenkachsen des Fingerelementes aufzubringen. Bei einer Ausgestaltung der Greifeinrichtung mit einem an einem Träger gelagerten Fingerelement ist bevorzugt der Freiheitsgrad um die Trägerschwenkachse gesperrt. Die Bewegung des ersten Fingerelementes um die Schwenkachse, die an dem Träger ausgebildet ist, die sogenannte Trägerschwenkachse, wird damit durch die Bewegung des Kraftübertragungselementes vorgegeben und bewirkt.

In einer alternativen Ausgestaltung ist es vorgesehen, dass das Kraftübertragungselement über ein Kugelgelenk mit dem ersten Fingerelement verbunden ist, wobei die Bewegung des ersten Fingerelementes, wie bei der kardanischen Lagerung auch, durch das Kraftübertragungselement gesteuert oder vorgegeben wird. Der Antrieb verändert die Winkelstellung des Kraftübertragungselementes in der Hauptebene der Antriebsbewegung, die im Wesentlichen senkrecht zu der Drehachse des Antriebes verläuft. Das Kraftübertragungselement wird in der Hauptebene verschenkt und steuert durch die Veränderung der Winkelstellung aufgrund der Drehbewegung die Bewegung des Fingerelementes. Das Kugelgelenk ermöglicht eine freie Verschwenkung des ersten Fingerelementes.

Das Kraftübertragungselement kann an einem drehbaren, von dem Antrieb angetriebenen Lagerzapfen gelagert sein. Der Lagerzapfen kann auf einer Kreisscheibe rotieren oder aber auf einem von dem Antrieb angetriebenen Arm gelagert sein. Wenn das Kraftübertragungselement, das bevorzugt als ein starres Bauteil, beispielsweise ein Rahmen, ein Zugkräfte und Druckkräfte übertragender Träger oder eine andere, im Wesentlichen starre Konstruktion, ausgebildet ist, um den Lagerzapfen verschwenkbar gelagert ist, führt das Kraftübertragungselement an der Lagerstelle eine kreisförmige oder eine kreisabschnittsförmige Bewegung durch oder bewegt sich auf einer kreisförmigen oder kreisabschnittsförmigen Trajektorie. Diese Bewegung findet bevorzugt in einer Ebene statt, wobei die horizontalen und vertikalen Bewegungsanteile einer Kreisbewegung in einer Ebene dazu dienen, eine zusammengesetzte Verlagerung des Fingerelementes um die beiden Schwenkachsen zu erreichen.

Das Kraftübertragungselement kann um eine Achse senkrecht zu der Längserstreckung des Lagerzapfens schwenkbar gelagert sein, so dass sich an der Lagerstelle an dem Lagerzapfen eine kardanische oder angenähert kardanische Lagerung des Kraftübertragungselementes ergibt. Dadurch können Blockaden in der Bewegung verhindert werden.

Wenn der Lagerzapfen an einem Arm angeordnet ist, kann dieser auf einem um eine Achse schwenkbaren Lagerarm an einem Rahmen gelagert sein, wodurch sich eine Überlagerung von Drehbewegungen ergibt, die dazu führt, dass die Orientierung des Kraftübertragungselementes in einer vielfältigen Art und Weise verändert werden kann, wodurch die Verschwenkung des Fingerelementes um die beiden Schwenkachsen aufeinander abgestimmt werden können.

Der Arm kann auf einer in dem Chassis gelagerten Drehscheibe angetrieben sein, wodurch eine leichte Realisierung des Antriebes durch Drehbewegungen erzielt werden kann.

Der Rahmen, an dem der Lagerarm gelagert und an dem somit auch der Arm und der Lagerzapfen gelagert ist, kann in einer Ebene auf dem Chassis verschieblich gelagert sein. Durch die Verschiebung des Rahmens in einer Ebene können weitere Fingerelemente gekoppelt mit dem ersten Fingerelement angetrieben werden. Die Verschieblichkeit kann über eine Kulissenführung beeinflusst werden, so dass komplexe Verlagerungsbewegungen des Rahmens und damit auch individuelle Bewegungen der weiteren Fingerelemente relativ zu dem Chassis möglich sind.

Eine Schwenkachse des ersten Fingerelementes kann die durch den Lagerzapfen gebildete Achse schneiden, so dass eine Drehung um diese Achse die Orientierung des ersten Fingerelementes bezüglich dieser einen Schwenkachse nicht beeinflusst.

Die zugkraftübertragende und druckkraftübertragende Ausgestaltung des Kraftübertragungselementes ermöglicht eine präzise Steuerung der Bewegung des ersten Fingerelementes in alle Richtungen, so dass eine direkte Kopplung zwischen dem Antrieb und dem Fingerelement ohne Federelemente möglich ist. Grundsätzlich ist es möglich, in der Kraftübertragungskette elastische Komponenten vorzusehen. Diese dienen hauptsächlich nicht der Rückstellung des Fingerelementes in eine Ausgangsposition, sondern zur Schonung der Mechanik oder zur Erreichung eines nachgiebigen Bewegungsverhaltens des Fingerelementes bei Einwirkung äußerer Kräfte, was insbesondere bei orthopädietechnischen Greifeinrichtungen für den Nutzer angenehm ist. Wird mit dem ersten Fingerelement gegen einen Gegenstand gestoßen, kann durch die Zwischenschaltung elastischer Pufferelemente eine direkte Krafteinleitung auf den Antrieb, den Motor und gegebenenfalls die Lagerung an einem Unterarmstumpf verringert werden.

Das zumindest eine zweite Fingerelement kann über einen um eine Achse klappbar an dem Chassis gelagerten Bügel mit dem Antrieb gekoppelt sein. Der Bügel erlaubt eine nahezu beliebige Anordnung einer Kraftübertragungseinrichtung, mit der der Bügel mit dem Antrieb gekoppelt werden kann. Dadurch lassen sich besonders leicht Einstellungen vornehmen, so dass Schwenkwinkel und Kraftübersetzungen durch eine unterschiedliche Positionierung der ersten Kraftübertragungseinrichtung zwischen dem Antrieb und dem Bügel verändert werden können. Die erste Kraftübertragungseinrichtung, die den Antrieb mit dem Bügel verbindet, kann an einem exzentrisch bewegten, mit dem Antrieb gekoppelten Lagerelement gelagert sein, so dass gemeinsam mit dem ersten Fingerelement bei einer Aktivierung des Antriebs der Bügel in die eine oder andere Richtung um die Achse schwenkbar bewegt oder geklappt werden kann.

Der Bügel kann über eine zweite Kraftübertragungseinrichtung mit dem zumindest einen zweiten Fingerelement gekoppelt sein. Die zweite Kraftübertragungseinrichtung kann an dem Bügel an unterschiedlichen Stellen festlegbar sein, so dass je nach Positionierung der zweiten Kraftübertragungseinrichtung an dem Bügel unterschiedliche Verstellwinkel relativ zu dem Chassis bei gleichem Verschwenkwinkel des Bügels relativ zu dem Chassis erreicht werden kann. Dadurch können die Bewegungen der zweiten Fingerelemente zueinander und der zweiten Fingerelemente relativ zu dem ersten Fingerelement verändert und auf die jeweilige Greifsituation oder den jeweiligen Einsatzzweck angepasst werden. Sowohl die erste Kraftübertragungseinrichtung als auch die zweite Kraftübertragungseinrichtung können zugkraftübertragend und druckkraftübertragend ausgebildet sein. Dadurch werden Rückstellelemente nicht benötigt, sondern eine Zwangsbewegung durch sich ändernde Drehrichtungen des Antriebes bewirkt.

Eine Weiterbildung der Erfindung sieht vor, dass der Bügel elastisch verformbar ausgebildet ist, wodurch eine Nachgiebigkeit der Fingerelemente gegenüber äußeren Kräften, also Kräften, die von dem Fingerelement auf das Chassis übertragen werden, gegeben ist. Ebenfalls ist es möglich, durch die elastische Verformbarkeit Veränderungen bei dem Verstellwinkel bzw. der jeweiligen Trajektorie zu erzeugen.

Alternativ zu dem Antrieb der zweiten Fingerelemente über den Bügel ist es möglich, den Rahmen als Ankopplungselement für die zweite Kraftübertragungseinrichtung zu verwenden, so dass über die zweite Kraftübertragungseinrichtung die Verlagerung des Rahmens relativ zu dem Chassis auf das jeweils über die zweite Kraftübertragungseinrichtung gekoppelte zweite Fingerelement zu übertragen.

Nachfolgend werden Ausführungsbeispiele der Erfindung anhand der beigefügten Figuren näher erläutert. Es zeigen:
Figur 1- eine Draufsicht auf eine geöffnete Greifeinrichtung in einer ersten Stellung;
Figur 2- eine Seitenansicht der Greifeinrichtung im Lateralgriff;
Figur 3- eine Ansicht gemäß Figur 1 in einer teilweise geschlossenen Stellung;
Figur 4- eine Ansicht gemäß Figur 3 in einer geschlossenen Stellung im Spitzgriff;
Figur 5- eine Ansicht der Stellung gemäß Figur 2 in Untenansicht;
Figur 6- eine Seitenansicht der Stellung gemäß Figur 4;
Figur 7- eine schematische Darstellung der Greifeinrichtung in einer Untenansicht;
Figur 8- eine schematische Darstellung gemäß Figur 7 in einer Seitenansicht;
Figur 9- eine Variante des Antriebes der zweiten Fingerelemente in Untenansicht;
Figur 10- eine Variante des Antriebs der zweiten Fingerelemente in Draufsicht; sowie
Figur 11- eine Seitenansicht der Figur 10.

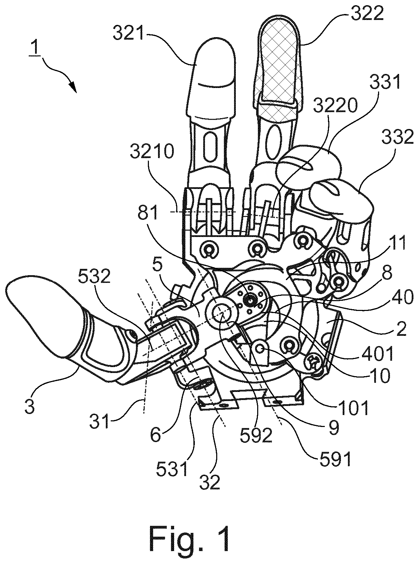

Figur 1 zeigt in einer Untenansicht eine Greifeinrichtung 1 in Gestalt einer Prothesenhand mit einem Chassis 2, an dem eine Schwalbenschwanzausnehmung ausgebildet ist, um das Chassis 2 mit proximalen Prothesenkomponenten, beispielsweise einem Unterarmschaft mechanisch zu verbinden. An dem Chassis 2 ist ein erstes Fingerelement 3 schwenkbar gelagert, das als Daumen ausgebildet ist. Weiterhin sind vier zweite Fingerelemente 321, 322, 331, 332 an dem Chassis 2 schwenkbar gelagert. Die beiden als Zeigefinger und Mittelfinger ausgebildeten zweiten Fingerelemente 321, 322 sind über jeweils eine Schwenkachse 3210, 3220 an dem Chassis 2 gelagert, die als Ringfinger und kleiner Finger ausgebildeten zweiten Fingerelemente 331, 332 sind ebenfalls über je eine, nicht erkennbare Schwenkachse3310, 3320 schwenkbar an dem Chassis 2 gelagert. Weiterhin ist an dem Chassis 2 ein motorischer Antrieb befestigt, der auf der Rückseite oder der dem Handrücken entsprechenden Seite der Greifeinrichtung 1 angeordnet ist. Über den Antrieb, der später erläutert werden wird, wird ein Kraftübertragungselement 5 angetrieben, das wiederrum mit dem ersten Fingerelement 3 gekoppelt ist, so dass bei einer Bewegung des Kraftübertragungselementes 5 das Fingerelement 3 relativ zu dem Chassis 2 und damit auch relativ zu den anderen Fingerelementen 321, 322, 331,332 verlagert wird. Das Chassis 2 bildet einen Rahmen, an dem die Fingerelemente befestigt und in dem der Antrieb und die anderen Komponenten gelagert sind. Das Chassis ist im Wesentlichen der Form einer Handfläche nachempfunden und weist eine Hauptebene auf, die im Wesentlichen der Handinnenfläche entspricht und in der Figur parallel zu der Blattebene orientiert ist.

Das Kraftübertragungselement 5 ist als starres Bauteil ausgebildet und H-förmig geformt. Antriebsseitig ist das Kraftübertragungselement 5 um zwei Achsen 591, 592 an einem Lagerzapfen 9 gelagert, der einen Exzenterpunkt der Drehscheibe 40 darstellt. Der Lagerarm 10 ist an einen Rahmen 11, der verschieblich an dem Chassis 2 gelagert ist, um eine Achse 101 schwenkbar befestigt. Der Lagerarm 10 ist an der Drehscheibe 40 befestigt und verschwenkbar um die Achse 81 gelagert. Um die Achse 81 ist ein weiterer Lagerzapfen gebildet, der einen weiteren Exzenterpunkt auf der Drehscheibe 40 bildet. Sowohl der Rahmen 11 als auch die Drehscheibe 40, der Lagerarm 10 und der Arm 8 sind im Wesentlichen parallel zueinander und parallel zu der Hauptebene des Chassis 2 angeordnet. Die Achse 101 des Schwenkarmes 10 läuft parallel zu der Schwenkachse 81 des Armes 8 und parallel zu der Achse 592, die durch die Längserstreckung des Zapfens 9 ausgebildet ist und um die das Kraftübertragungselement 5 drehbar gelagert ist. Die Lagerung des Kraftübertragungselementes 5 erfolgt über eine Hülse, an der Zapfen angeordnet sind, die eine zweite Achse 591 ausbilden, die im Wesentlichen orthogonal zu der Achse 592, die durch die Längserstreckung des Lagerzapfens 9 ausgebildet ist, orientiert ist. Die beiden Achsen 591, 592 schneiden sich. Die zweite Achse 591 verläuft im Wesentlichen parallel zu der Ebene, in der der Arm 8, der Lagerarm 10 oder die Drehscheibe 40 angeordnet sind.

Das der Antriebsseite gegenüberliegende Ende des Kraftübertragungselementes 5 ist an dem ersten Fingerelement 3 um eine erste Achse 531 schwenkbar gelagert, die im Wesentlichen parallel zu der zweiten Achse 591 an dem Lagerzapfen 9 orientiert ist. Das erste Fingerelement 3 ist über einen Träger 6 an dem Chassis 2 gelagert. Der Träger 6 ist schwenkbar um eine Schwenkachse 32 an dem Chassis 2 gelagert und bildet an dem dem Chassis 2 abgewandten Ende eine zweite Schwenkachse 31 aus, die anders als die chassisseitige Schwenkachse 32 orientiert ist. Das erste Fingerelement 3 kann somit relativ zu dem Chassis 2 um zwei Schwenkachsen 31, 32 verlagert werden, die durch den Träger 6 ausgebildet sind. Die chassisseitige Schwenkachse 32 ist so orientiert, dass das erste Fingerelement 3 nach außen verschwenkt oder extendiert werden kann, so dass das erste Fingerelement 3 in Richtung auf eine Ebene verschwenkt wird, in der sich der Rahmen 11 oder die Drehscheibe 40 befindet. Bei einer Ausgestaltung der Greifeinrichtung als Prothesenhand entspricht diese Ebene der Handinnenfläche. In die andere Richtung verschwenkt oder flektiert bewegt sich das erste Fingerelement 3 um die chassisseitige Schwenkachse 32 in Richtung auf die gegenüberliegenden zweiten Fingerelemente 331, 332, also im Wesentlichen senkrecht zu der Blattebene gemäß der Figur 1.

Die fingerseitige Schwenkachse 31 schneidet die chassisseitige Schwenkachse 32 in dem dargestellten Ausführungsbeispiel nicht und verläuft in einer Ebene, die orthogonal zu der chassisseitigen Schwenkachse 32 orientiert ist. Dadurch ist es möglich, dass das erste Fingerelement 3 um die fingerseitige Schwenkachse 31 verschwenkt werden kann, wobei die Verschwenkbewegung in Richtung auf den Zeigefinger 321 oder davon weg erfolgen kann. Damit dies bei einem starren Kraftübertragungselement 5 möglich ist, ist das Kraftübertragungselement 5 zusätzlich zu der ersten Achse 531 um eine zweite Achse 532 an dem Fingerelement 3 gelagert, so dass sich eine kardanische Lagerung ergibt, die in einem rotatorischen Freiheitsgrad gesperrt ist, um eine Kraftübertragung zur Verschwenkung des Fingerelementes um die fingerseitige Schwenkachse 31 zu bewirken, wenn das Kraftübertragungselement 5 von dem Antrieb bewegt wird.

Wird der Arm 8 um eine Drehachse 401 der Drehscheibe 40 entgegen dem Uhrzeigersinn verdreht und der Lagerarm 10 im Uhrzeigersinn um die Achse 101 verschwenkt, bewegt sich das Kraftübertragungselement 5 nach rechts. Die Achse 591 verschwenkt in Uhrzeigerrichtung um den Lagerzapfen 9, und das erste Fingerelement 3 wird um die chassisseitige Schwenkachse 32 in Richtung auf die Handinnenfläche oder auf das gegenüberliegende zweite Fingerelement 332, das bei einer Prothesenhand dem kleinen Finger entspricht, verlagert. Eine umgekehrte Bewegung und eine Verlagerung der Achse 591 in Richtung auf die chassisseitige Schwenkachse 32, also nach links, würde eine Verschwenkung um die chassisseitige Schwenkachse 32 und damit ein Öffnen der Greifeinrichtung 1 oder ein Bewegen des ersten Fingerelementes 31 nach außen bewirken. Gleichzeitig wird aufgrund der Veränderung der Orientierung des Lagerzapfens 9 relativ zu der fingerseitigen Schwenkachse 31 eine Verschwenkbewegung des Fingerelementes 3 um die fingerseitige Schwenkachse 31 bewirkt. Da das erste Fingerelement 3 nicht parallel oder koaxial zu der fingerseitigen Schwenkachse 31 verläuft, sondern davon abragt, also die Längserstreckung des ersten Fingerelementes nicht mit der Schwenkachse 31 übereinstimmt, verändert sich dadurch auch die Orientierung des ersten Fingerelements 3 zu dem Chassis 2 und die Entfernung und die Winkelstellung zu den anderen zweiten Fingerelementen 321, 322, 331, 332. Ausgehend von der maximal geöffneten oder extendierten Stellung, die in der Figur 1 angenähert erreicht ist, ergeben sich je nach Drehrichtung des Antriebes und Verschwenkbewegung des Lagerzapfens 9 in Verbindung mit der Verschwenkung des Armes 8 oder des Lagerarmes 10 unterschiedliche Trajektorien und Bewegungsverläufe der jeweiligen Fingerelemente 3, 321, 322, 331, 332 zueinander.

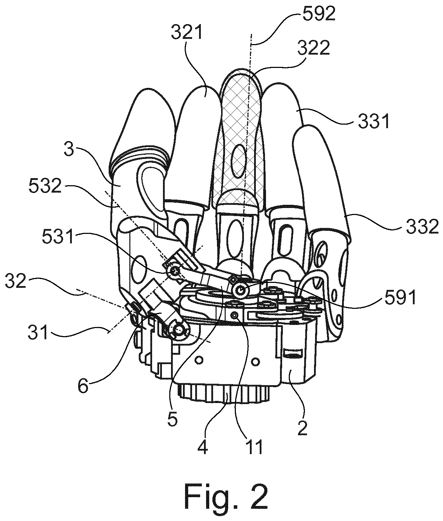

Figur 2 zeigt eine Seitenansicht aus Richtung der proximalen Anschlussstelle, also im Bereich der Schwalbenschwanzführung zur Befestigung der Greifeinrichtung 1 an einer weiteren Komponente, beispielsweise einem Unterarmschaft. Auf der dem Kraftübertragungselement 5 abgewandten Seite des Chassis 2 ist der Antrieb 4 in Gestalt eines Elektromotors zu erkennen. Über den Antrieb 4 werden gegebenenfalls unter Zwischenschaltung eines Getriebes die Drehscheibe 40 und damit der Lagerarm 10 und Arm 8 sowie der Rahmen 11 innerhalb des Chassis 2 verlagert. Der Figur 2 ist die in parallelen Ebenen geschichtete Anordnung des Rahmens 11 mit der darin angeordneten Drehscheibe 8 und dem Lagerarm 10 und dem darüber angeordneten Arm 8 zu erkennen. Die Achse 591 verläuft im Wesentlichen parallel zu der Ebene des Rahmens 11, wohingegen die Zweitachse 592 senkrecht zu dieser Ebene orientiert ist und der Längserstreckung des Lagerzapfens 9 entspricht. Die chassisseitige Schwenkachse 32 verläuft ebenfalls im Wesentlichen parallel zu der Ebene des Rahmens 11, so dass der Träger 6 mit seinen zwei senkrecht zueinander orientierten, zueinander beabstandeten Schwenkachsen 32, 31 in eine Ebene parallel zu der Ebene des Rahmens 11 oder senkrecht dazu um die chassisseitige Schwenkachse 32 verschwenkt werden kann. Das Fingerelement 3 kann zusätzlich um die fingerseitige Schwenkachse 31 verschwenkt werden. Der Figur 2 ist weiterhin die Ausrichtung der ersten Achse 531 zu entnehmen, die sich in dieser beispielhaften Ausführungsform parallel zu der Achse 591 durch die beiden Zapfen senkrecht zu dem Lagerzapfen 9 erstreckt. Zur Erzeugung einer kardanischen Lagerung ist die Achse 531 über einen um die zweite Achse 532 schwenkbaren Lagerkörper kardanisch gelagert. Figur 2 zeigt die Stellung der Greifeinrichtung in einem sogenannten Lateralgriff, bei dem das erste Fingerelement 3 seitlich an das nächstliegende zweite Fingerelement 321 angelegt ist, was beispielsweise zum Greifen flächiger Elemente wie einem Blatt oder dergleichen benötigt wird. Eine solche Stellung der Fingerelemente 3, 321 wird erreicht, indem der Antrieb 4 so bewegt wird, dass ausgehend von der geöffneten, extendierten Stellung gemäß Figur 1 die zweiten Fingerelemente 321, 322 schneller aus der durch den Rahmen 11 gebildeten Ebene oder Hauptebene des Chassis 2 nach oben verschwenkt werden als das erste Fingerelement 3, das darüber hinaus nach vorne verschwenkt wird, also um die fingerseitige Schwenkachse 31.

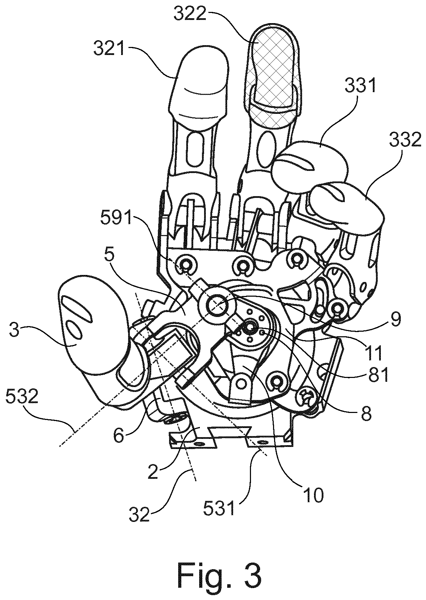

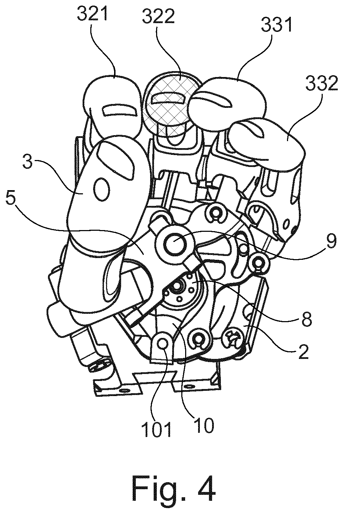

Figur 3 zeigt eine Stellung der Fingerelemente 3, 321, 322, 331, 332, bei der der Lagerzapfen 9 ausgehend von der Ausgangsstellung gemäß Figur 1 im Uhrzeigersinn verschwenkt wurde. Ebenfalls wurde die Drehachse 81 des Armes 8 im Uhrzeigersinn um die Drehachse 401 der Drehscheibe 40 verlagert. Der Lagerarm 10 wird durch die Verlagerung der Drehchse 81 nach unten bewegt. Dadurch wird auch der Rahmen 11 durch die Lagerung um die Achse 101 im Uhrzeigersinn verschwenkt und damit nach unten bewegt. Dadurch werden die zweiten Fingerelemente 321, 322, 331, 332 um ihre jeweiligen Schwenkachsen verschwenkt, sodass die Greifeinrichtung zunehmend geschlossen wird, wenn die Fingerelemente 3, 321, 322, 331, 332 flektiert werden. Das erste Fingerelement 3 wurde um die fingerseitige Schwenkachse 31 entgegen dem Uhrzeigersinn verdreht, gleichzeitig fand eine Verschwenkung um die chassisseitige Schwenkachse 32 in Richtung auf die anderen zweiten Fingerelemente 321, 322, 331, 332 statt, sodass die Achse 531 entgegen dem Uhrzeigersinn verdreht wurde und bei einer weiteren Verlagerung des Lagerzapfens 9 das erste Fingerelement 3 mit den beiden zweiten Fingerelementen 321, 322 zusammengeführt wird. Diese Weiterbewegung und die zusammengeführte Stellung ist in der Figur 4 gezeigt.

Der Rahmen 11 ist weiter nach unten verlagert und der Lagerarm 10 um die Achse 101 weiter im Uhrzeigersinn verdreht, ebenso der Arm 8, so dass der Lagerzapfen 9 weiter nach rechts entlang einer Bewegungsbahn verlagert ist. Die distalen Enden der Fingerelemente 3, 321 berühren sich, eine weitere Verlagerung des Lagerzapfens 9 würden zu einer erhöhten Anpresskraft der Fingerelemente 3, 321 aneinander führen. Die in der Figur 4 gezeigte Stellung ist der sogenannte Spitzgriff, bei der die Spitzen der gekrümmt ausgebildeten Fingerelemente 3, 321, 322 aneinander anliegen.

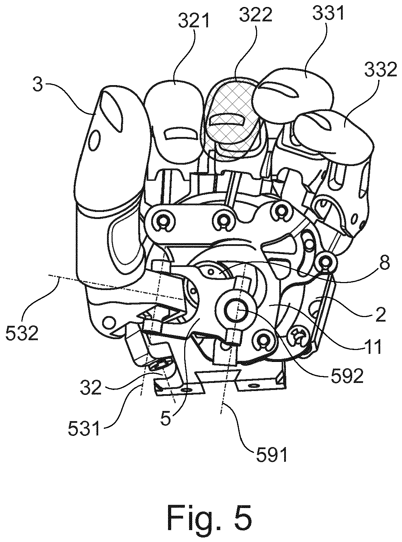

Demgegenüber ist die alternative Endstellung, bei der Lagerzapfen 9 entgegen dem Uhrzeigersinn maximal verdreht ist, in der Figur 5 gezeigt. Der sogenannte Lateralgriff führt das erste Fingerelement 3 seitlich an das zweite Fingerelement 321, der Rahmen 11 ist im Vergleich mit der Figur 4 weiter nach unten verschoben, so dass die zweiten Fingerelemente 321, 322, 331, 332 weiter aus der Hauptebene des Chassis 2 heraus in eine geschlossene Stellung verschwenkt werden, bevor ein Kontakt mit dem ersten Fingerelement 3 erreicht ist.

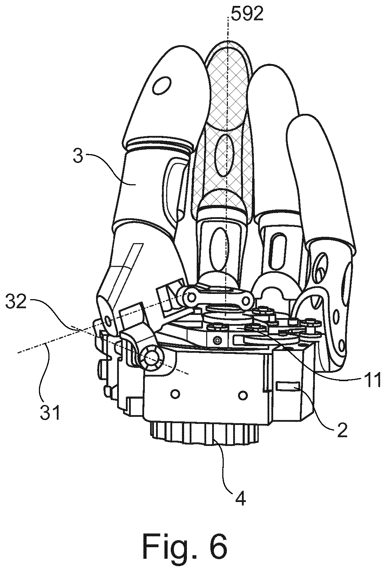

Der Spitzgriff gemäß Figur 4 ist in der Figur 6 in einer Seitenansicht gezeigt, alle Fingerelemente 3, 321,322, 331, 332 sind maximal nach oben aus der Ebene des Rahmens 11 oder der Hauptfläche oder Handinnenfläche in eine flektierte Stellung verschwenkt.

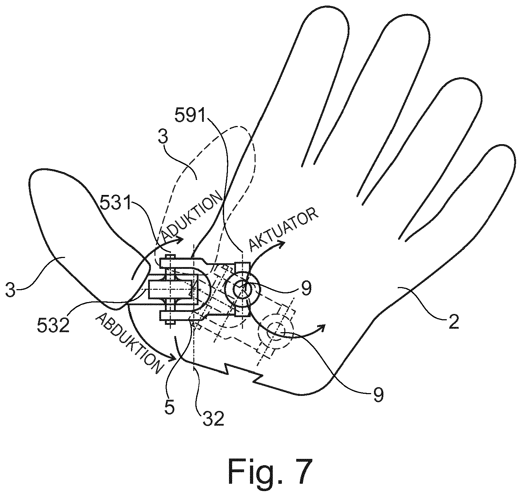

Figur 7 zeigt eine schematische Darstellung der Greifeinrichtung in der Position gemäß Figur 1 in den durchgezogenen Linien, in der gestrichelten Darstellung in der Position gemäß Figur 5. Die Lagerung, der Antrieb und die Zuordnung der zweiten Fingerelemente sind hier nicht dargestellt. Ausgehend von der maximal geöffneten Stellung der Greifeinrichtung wird das Kraftübertragungselement 5 entgegen dem Uhrzeigersinn entlang einer Kreisbahn verschwenkt, so dass der Lagerzapfen 9 auf der Kreisbahn nach unten und nach rechts wandert. Durch den Bewegungsanteil nach rechts verschwenkt das erste Fingerelement 3 um die chassisseitige Schwenkachse 32 und führt eine Schließbewegung durch, durch den Bewegungsanteil nach unten erfolgt die Verschwenkung um die fingerseitige Schwenkachse 31, wodurch eine Adduktion des ersten Fingerelementes 3, also ein Heranziehen an das Chassis 2 und die zweiten Fingerelemente erfolgt. Die Endstellung, wie sie in der gestrichelten Darstellung gezeigt ist, entspricht der in Figur 5 gezeigten Stellung des Lateralgriffes. Bei einer Verschwenkung durch den Antrieb 4 in entgegengesetzter Richtung, also in Uhrzeigerrichtung, wird der Lagerzapfen 9 nach oben und nach rechts verlagert, wodurch sich neben der Schließbewegung um die chassisseitige Schwenkachse 32 eine Abduktionsbewegung um die fingerseitige Schwenkachse 31 einstellt, so dass sich in der jeweiligen Endstellung der Spitzgriff einstellt, wie in der Figur 4 gezeigt ist.

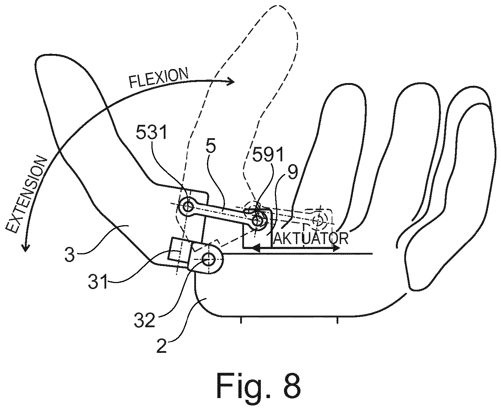

In der Figur 8 ist in einer Seitenansicht die Bewegung des ersten Fingerelementes 3 relativ zu dem Chassis 2 gezeigt. Bei einer Verschwenkung ausgehend von der Ausgangsstellung gemäß Figur 7 wird durch die Bewegungskomponente von der chassisseitgen Schwenkachse 32 weg eine Flexion des ersten Fingerelementes 3 relativ zu dem Chassis 2 bewirkt, während bei einer entgegengesetzten Bewegung eine Extension, also eine Vergrößerung des Winkels zwischen dem ersten Fingerelement 3 auf Seiten des Kraftübertragungselementes 5 relativ zu dem Chassis 2 durchgeführt wird. Die flektierte Stellung ist in der Strichlinie gezeigt, die extendierte Stellung in der durchgezogenen Linienführung. Die fingerseitige erste Achse 531 des Kraftübertragungselementes 5 liegt oberhalb der zweiten Achse 591, um die herum schwenkbar das Kraftübertragungselement 5 an dem Lagerzapfen 9 gelagert ist. Durch eine Verschiebung der Achse 591 in der Hauptebene ist eine Verschiebung der Achse 531 und damit eine Verschwenkung des Trägers 6 und des daran angeordneten Fingerelementes 3 um die chassisseitige Schwenkachse 32 möglich.

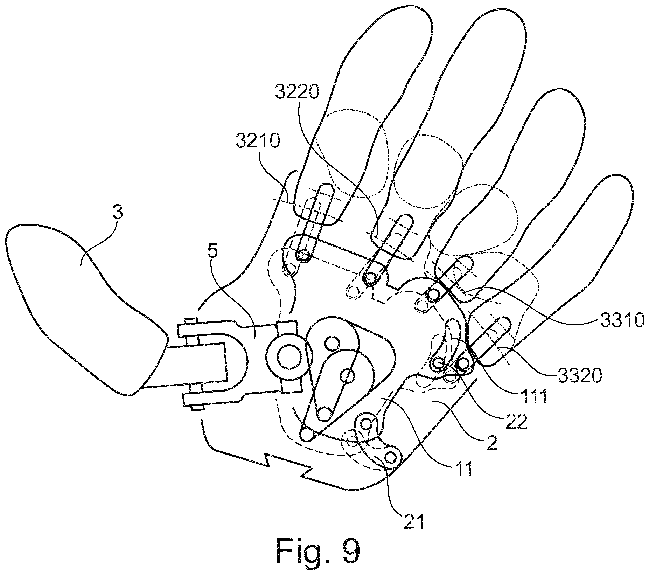

Figur 9 zeigt bei einer schematischen Darstellung die Bewegung der zweiten Fingerelemente 321, 322, 331, 332 durch die Verlagerung des Rahmens 11 relativ zu dem Chassis 2. Über den Antrieb wird der Rahmen 11 in einer gebogenen Kulisse 111 bewegt, in der ein Zapfen 22 des Chassis geführt ist. Weiterhin ist ein Schwenkarm 21 an dem Chassis 2 gelagert, der schwenkbar mit dem Rahmen 11 im Bereich der Befestigungsstelle der Greifeinrichtung gelagert ist. Wird der Rahmen 11 ausgehend von der Ausgangsstellung, die in den durchgezogenen Linien dargestellt ist, durch den Antrieb 4 nach unten verlagert, bewegt sich der Rahmen 11 entlang der Kulisse 111 und um den Schwenkarm 21 herum in die Stellung, die in Strichlinien gezeigt ist. Die zweiten Fingerelemente 321, 322, 331, 332 verschwenken um die jeweiligen Schwenkachsen 3210, 3220, 3310, 3320, da der Rahmen 11 über zweite Kraftübertragungselemente 51 mit den zweiten Fingerelementen 321, 322, 331, 332 gekoppelt ist.

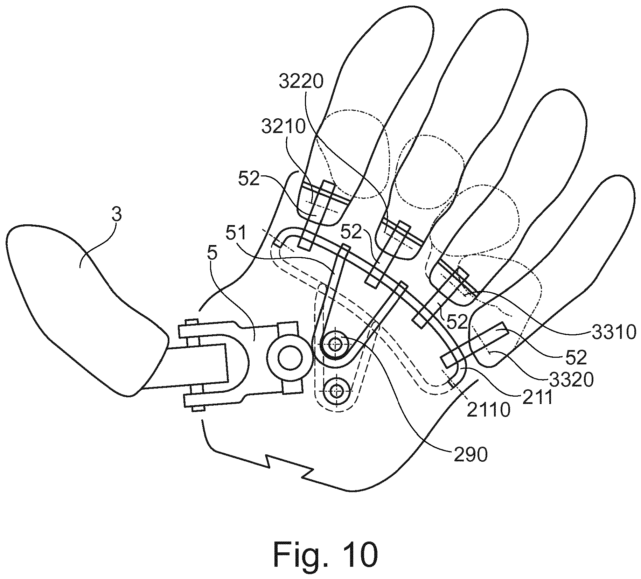

Eine alternative Art und Weise des Antriebes der zweiten Fingerelemente ist in der Figur 10 dargestellt. Statt eines Rahmens 11 ist an dem Chassis 2 ein Bügel 211 um eine Schwenkachse 2110 gelagert. Der Bügel 211 kann aus der dargestellten extendierten Stellung der zweiten Fingerelemente 321, 322, 331, 332 in eine flektierte Stellung um die Achse 2110 verlagert werden. Die zweiten Fingerelemente 321, 322, 331, 332 befinden sich dann in einer geschlossenen Stellung, der Bügel 211 ist von den Schwenkachsen 3210, 3220, 3310, 3320 weg verschwenkt, wie es in der gestrichelten Darstellung gezeigt ist. Um diese Verschwenkbewegung des Bügels 211 auszuführen, ist der Bügel 211 über eine erste Kraftübertragungseinrichtung 51 mit dem Antrieb 4 verbunden. Die erste Kraftübertragungseinrichtung 51 ist auf einem exzentrisch gelagerten, mit dem Antrieb 4 gekoppelten Lagerelement 290 oder einem Zapfen gelagert. Im dargestellten Ausführungsbeispiel ist die Kraftübertragungseinrichtung 51 als eine Schlaufe ausgebildet, die um das Lagerelement 290 herumgelegt ist. Die erste Kraftübertragungseinrichtung 51 kann an dem Lagerelement 290 entlanggleiten, so dass bei der ausgeführten Kreisbewegung des Lagerelementes 290 bei einer Verdrehung einer Position maximal nahe der Achse 2110 zu einer Position maximal entfernt der Achse 2110 entlang einer Kreisbahn ein Längenausgleich zwischen den beiden Abschnitten der Kraftübertragungseinrichtung erfolgt. Die Kraftübertragungseinrichtung 51 kann verschwenkbar und gegebenenfalls verschieblich an dem Bügel 211 gelagert sein. Der Bügel 211 kann aus einem elastischen Material, beispielsweise einem elastischen Kunststoffmaterial ausgebildet sein.

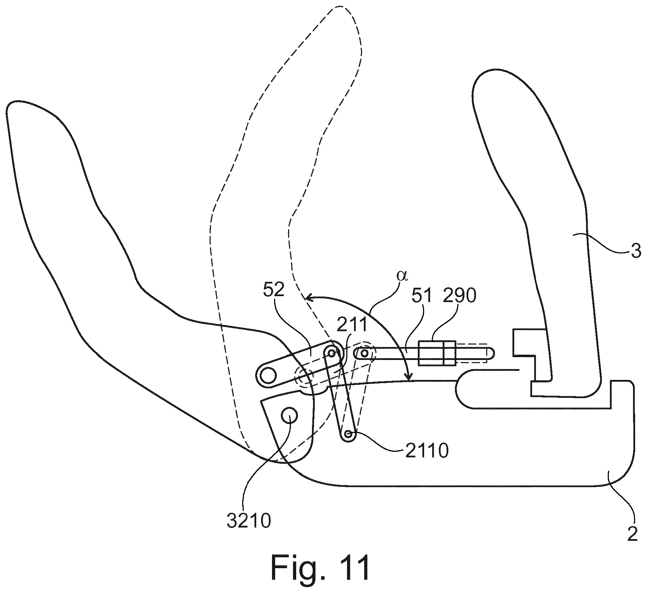

Figur 11 zeigt die Betätigung der zweiten Fingerelemente mittels des Bügels 211 in einer Seitenansicht. In der Ausgangsstellung sind die zweiten Fingerelemente extendiert, das heißt, dass der Winkel α zwischen der Hauptebene des Chassis 2 und der der Kraftübertragungseinrichtung 51 zugewandten Seite des zweiten Fingerelementes maximal ist. In der gestrichelten Darstellung ist der eingeschlossene Winkel α minimal, das heißt, dass die zweiten Fingerelemente nahezu senkrecht zu der Hauptebene des Chassis 2 stehen. Das Lagerelement 290 ist maximal entfernt von der Achse 2110 des Bügels 211 verlagert, so dass über die Verlagerung des Lagerelementes 290 und die Verschwenkung des Bügels 211 und der damit einhergehenden Verschwenkung der zweiten Kraftübertragungseinrichtung, die den Bügel 211 mit den jeweiligen zweiten Fingerelementen koppeln, die Verschwenkung des jeweiligen zweiten Fingerelementes um die jeweilige Schwenkachse an dem Chassis erfolgt.

## Patentansprüche

1. Greifeinrichtung für eine Handprothese mit
- einem Chassis (2),
- zumindest einem an dem Chassis (2) schwenkbar gelagerten ersten Fingerelement (3),
- zumindest einem Drehbewegung erzeugenden Antrieb (4) und
- einem Kraftübertragungselement (5), das den Antrieb (4) mit dem ersten Fingerelement (3) koppelt, wobei das Kraftübertragungselement (5) angepasst ist, um das erste Fingerelement (3) um zwei unterschiedlich orientierte Schwenkachsen (31, 32) relativ zu dem Chassis (2) zu verschwenken, wobei zumindest ein zweites Fingerelement (321, 322, 323, 324) schwenkbar an dem Chassis (2) gelagert und angepasst ist, um bei einer Aktivierung des Antriebes (4) zu verschwenken und mit dem Antrieb (4) dergestalt gekoppelt ist, dass ausgehend von einer Ruhestellung in Abhängigkeit von der Drehrichtung des Antriebes (4) das erste und zweite Fingerelement (3; 321, 322, 323, 324) unterschiedliche Trajektorien relativ zueinander durchlaufen.

2. Greifeinrichtung nach Anspruch 1, **dadurch gekennzeichnet, dass** die Schwenkachsen (31, 32) senkrecht zueinander orientiert sind und/oder sich nicht schneiden.

3. Greifeinrichtung nach Anspruch 1 oder 2, **dadurch gekennzeichnet, dass** das erste Fingerelement (3) an einem Träger (6) schwenkbar um eine der Schwenkachsen (31) gelagert ist und der Träger (6) an dem Chassis (2) um die andere Schwenkachse (32) schwenkbar gelagert ist.

4. Greifeinrichtung nach Anspruch 3, **dadurch gekennzeichnet, dass** das Kraftübertragungselement (5) an dem ersten Fingerelement (3) befestigt ist.

5. Greifeinrichtung nach Anspruch 4, **dadurch gekennzeichnet, dass** das Kraftübertragungselement (5) schwenkbar an dem ersten Fingerelement (3) gelagert ist.

6. Greifeinrichtung nach Anspruch 5, **dadurch gekennzeichnet, dass** das Kraftübertragungselement (5) kardanisch ober über ein Kugelgelenk an dem ersten Fingerelement (3) gelagert ist und die Bewegung des ersten Fingerelementes (3) um eine der Schwenkachsen (31) durch das Kraftübertragungselement (5) gesteuert wird.

7. Greifeinrichtung nach einem der voranstehenden Ansprüche, **dadurch gekennzeichnet, dass** das Kraftübertragungselement (5) an einem drehbaren, von dem Antrieb (4) angetriebenen Arm (8) um einen Lagerzapfen (9) schwenkbar gelagert ist.

8. Greifeinrichtung nach Anspruch 7, **dadurch gekennzeichnet, dass** das Kraftübertragungselement (5) um eine Achse (591) senkrecht zu der Längserstreckung des Lagerzapfens (9) schwenkbar gelagert ist.

9. Greifeinrichtung nach Anspruch 7, **dadurch gekennzeichnet, dass** der Arm (8) auf einem um eine Achse (101) schwenkbaren Lagerarm (10) an einem Rahmen (11) gelagert ist.

10. Greifeinrichtung nach Anspruch 7 oder 9, **dadurch gekennzeichnet, dass** der Arm (8) von einer in dem Chassis (2) gelagerten Drehscheibe (40) angetrieben ist.

11. Greifeinrichtung nach Anspruch 9 oder 10, **dadurch gekennzeichnet, dass** der Rahmen (11) in einer Ebene auf dem Chassis (2) verschieblich gelagert ist.

12. Greifeinrichtung nach einem Ansprüche 7 bis 11, **dadurch gekennzeichnet, dass** eine Schwenkachse (31) des ersten Fingerelementes (3) die durch den Lagerzapfen (9) gebildete Achse (592) schneidet.

13. Greifeinrichtung nach einem der voranstehenden Ansprüche, **dadurch gekennzeichnet, dass** das Kraftübertragungselement (5) zug- und druckkraftübertragend ausgebildet ist.

14. Greifeinrichtung nach Anspruch 1, **dadurch gekennzeichnet, dass** zumindest eine zweite Fingerelement (321, 322, 323, 324) über einen um eine Achse (2110) klappbar an dem Chassis (2) gelagerten Bügel (211) mit dem Antrieb (4) gekoppelt ist.

15. Greifeinrichtung nach Anspruch 14, **dadurch gekennzeichnet, dass** der Bügel (211) über eine erste Kraftübertragungseinrichtung (51) mit dem Antrieb (4) verbunden ist.

16. Greifeinrichtung nach Anspruch 15, **dadurch gekennzeichnet, dass** die erste Kraftübertragungseinrichtung (51) an einem exzentrisch bewegten, mit dem Antrieb (4) gekoppelten Lagerelement (290) gelagert ist.

17. Greifeinrichtung nach einem der Ansprüche 14 bis 16, **dadurch gekennzeichnet, dass** der Bügel (211) über eine zweite Kraftübertragungseinrichtung (52) mit dem zumindest einen zweiten Fingerelement (321, 322, 323, 324) gekoppelt ist.

18. Greifeinrichtung nach einem der Ansprüche 14 bis 17, **dadurch gekennzeichnet, dass** der Bügel (211) elastisch verformbar ausgebildet ist.

19. Orthopädietechnische Greifeinrichtung nach Anspruch 1 in Verbindung mit Anspruch 9, 10 oder 11, **dadurch gekennzeichnet, dass** der Rahmen (11) mit dem zweiten Fingerelement (321, 322) gekoppelt ist.

## Claims

1. A gripping device for a hand prosthesis having
- a chassis (2),
- at least one first finger element (3) mounted pivotably on the chassis (2),
- at least one a rotationl movement producing drive (4), and
- a force transmission element (5) coupling the drive (4) to the first finger element (3), whereas the force transmission element (5) is adapted to pivot the first finger element (3) relative to the chassis (2) about two differently oriented pivot axes (31, 32), wherein at least one second finger element (321, 322, 323, 324) is mounted pivotably on the chassis (2) and is adapted to pivot upon activation of the drive (4) and is coupled to the drive (4) in such a way that, proceeding from a rest position, and depending on the direction of rotation of the drive (4), the first and second finger elements (3; 321, 322, 323, 324) travel on different trajectories relative to each other.

2. The gripping device as claimed in claim 1, **characterized in that** the pivot axes (31, 32) are oriented perpendicularly with respect to each other and/or do not intersect each other.

3. The gripping device as claimed in claim 1 or 2, **characterized in that** the first finger element (3) is mounted on a support (6) pivotably about one of the pivot axes (31), and the support (6) is mounted pivotably on the chassis (2) about the other pivot axis (32).

4. The gripping device as claimed in claim 3, **characterized in that** the force transmission element (5) is secured on the first finger element (3).

5. The gripping device as claimed in claim 4, **characterized in that** the force transmission element (5) is mounted pivotably on the first finger element (3).

6. The gripping device as claimed in claim 5, **characterized in that** the force transmission element (5) is mounted on the first finger element (3) by a cardan joint or by a ball-and-socket joint, and the movement of the first finger element (3) about one of the pivot axes (31) is controlled by the force transmission element (5).

7. The gripping device as claimed in one of the preceding claims, **characterized in that** the force transmission element (5) is mounted pivotably about a bearing pin (9) on a rotatable arm (8) driven by the drive (4).

8. The gripping device as claimed in claim 7, **characterized in that** the force transmission element (5) is mounted pivotably about an axis (591) perpendicular to the longitudinal extent of the bearing pin (9).

9. The gripping device as claimed in claim 7, **characterized in that** the arm (8) is mounted at a frame (11) on a bearing arm (10) pivotable about an axis (101).

10. The gripping device as claimed in claim 7 or 9, **characterized in that** the arm (8) is driven by a rotary disk (40) mounted in the chassis (2).

11. The gripping device as claimed in claim 9 or 10, **characterized in that** the frame (11) is mounted displaceably in one plane on the chassis (2).

12. The gripping device as claimed in one of claims 7 through 11, **characterized in that** a pivot axis (31) of the first finger element (3) intersects the axis (592) formed by the bearing pin (9).

13. The gripping device as claimed in one of the preceding claims, **characterized in that** the force transmission element (5) is configured to transmit tensile force and compressive force.

14. The gripping device as claimed in claim 1, **characterized in that** at least one second finger element (321, 322, 323, 324) is coupled to the drive (4) via a bracket (211) mounted on the chassis (2) so as to be foldable about an axis (2110).

15. The gripping device as claimed in claim 14, **characterized in that** the bracket (211) is connected to the drive (4) via a first force transmission device (51).

16. The gripping device as claimed in claim 15, **characterized in that** the first force transmission device (51) is mounted on an eccentrically moved bearing element (290) that is coupled to the drive (4).

17. The gripping device as claimed in one of claims 14 through 16, **characterized in that** the bracket (211) is coupled to the at least one second finger element (321, 322, 323, 324) via a second force transmission device (52).

18. The gripping device as claimed in one of claims 14 through 17, **characterized in that** the bracket (211) is designed to be elastically deformable.

19. The orthopedic gripping device as claimed in claim 1 in conjunction with claim 9, 10 or 11, **characterized in that** the frame (11) is coupled to the second finger element (321, 322).

## Revendications

1. Dispositif de préhension pour une main prothétique, comprenant
- un châssis (2),
- au moins un premier élément de doigt (3) monté sur le châssis (2) de manière à pouvoir pivoter,
- au moins un entraînement (4) engendrant un mouvement de rotation, et
- un élément de transmission de force (5) qui couple l'entraînement (4) au premier élément de doigt (3), l'élément de transmission de force (5) étant adapté pour faire pivoter le premier élément de doigt (3) par rapport au châssis (2) autour de deux axes de pivotement (31, 32) orientés différemment,
dans lequel au moins un deuxième élément de doigt (321, 322, 323, 324) est monté sur le châssis (2) de manière à pouvoir pivoter et est adapté pour pivoter lors d'une activation de l'entraînement (4) et est couplé à l'entraînement (4) de telle sorte qu'à partir d'une position de repos, en fonction du sens de rotation de l'entraînement (4), les premier et deuxième éléments de doigt (3 ; 321, 322, 323, 324) suivent des trajectoires différentes l'une de l'autre.

2. Dispositif de préhension selon la revendication 1,
**caractérisé en ce que** les axes de pivotement (31, 32) sont orientés perpendiculairement l'un à l'autre et/ou ne se coupent pas.

3. Dispositif de préhension selon la revendication 1 ou 2,
**caractérisé en ce que** le premier élément de doigt (3) est monté sur un support (6) de manière à pouvoir pivoter autour de l'un des axes de pivotement (31), et le support (6) est monté sur le châssis (2) de manière à pouvoir pivoter autour de l'autre axe de pivotement (32).

4. Dispositif de préhension selon la revendication 3,
**caractérisé en ce que** l'élément de transmission de force (5) est fixé au premier élément de doigt (3).

5. Dispositif de préhension selon la revendication 4,
**caractérisé en ce que** l'élément de transmission de force (5) est monté sur le premier élément de doigt (3) de manière à pouvoir pivoter.

6. Dispositif de préhension selon la revendication 5,
**caractérisé en ce que** l'élément de transmission de force (5) est monté sur le premier élément de doigt (3) à la Cardan ou par l'intermédiaire d'une articulation à rotule, et le mouvement du premier élément de doigt (3) autour de l'un des axes de pivotement (31) est commandé par l'élément de transmission de force (5).

7. Dispositif de préhension selon l'une des revendications précédentes,
**caractérisé en ce que** l'élément de transmission de force (5) est monté sur un bras rotatif (8), entraîné par l'entraînement (4), de manière à pouvoir pivoter autour d'un tourillon de montage (9).

8. Dispositif de préhension selon la revendication 7,
**caractérisé en ce que** l'élément de transmission de force (5) est monté de manière à pouvoir pivoter autour d'un axe (591) perpendiculaire à l'extension longitudinale du tourillon de montage (9).

9. Dispositif de préhension selon la revendication 7,
**caractérisé en ce que** le bras (8) est monté sur un cadre (11) au niveau d'un bras de montage (10) pouvant pivoter autour d'un axe (101).

10. Dispositif de préhension selon la revendication 7 ou 9,
**caractérisé en ce que** le bras (8) est entraîné par un disque rotatif (40) monté dans le châssis (2).

11. Dispositif de préhension selon la revendication 9 ou 10,
**caractérisé en ce que** le cadre (11) est monté coulissant dans un plan sur le châssis (2).

12. Dispositif de préhension selon l'une des revendications 7 à 11,
**caractérisé en ce qu'**un axe de pivotement (31) du premier élément de doigt (3) coupe l'axe (592) formé par le tourillon de montage (9).

13. Dispositif de préhension selon l'une des revendications précédentes,
**caractérisé en ce que** l'élément de transmission de force (5) est réalisé de manière à transmettre des forces de traction et de compression.

14. Dispositif de préhension selon l'une des revendications précédentes,
**caractérisé en ce qu'**au moins un deuxième élément de doigt (321, 322, 323, 324) est couplé à l'entraînement (4) par l'intermédiaire d'un étrier (211) monté sur le châssis (2) de manière à pouvoir être rabattu autour d'un axe (2110).

15. Dispositif de préhension selon la revendication 14,
**caractérisé en ce que** l'étrier (211) est relié à l'entraînement (4) par l'intermédiaire d'un premier dispositif de transmission de force (51).

16. Dispositif de préhension selon la revendication 15,
**caractérisé en ce que** le premier dispositif de transmission de force (51) est monté sur un élément de montage (290) à mouvement excentrique, couplé à l'entraînement (4).

17. Dispositif de préhension selon l'une des revendications 14 à 16,
**caractérisé en ce que** l'étrier (211) est couplé audit au moins un deuxième élément de doigt (321, 322, 323, 324) par l'intermédiaire d'un deuxième dispositif de transmission de force (52).

18. Dispositif de préhension selon l'une des revendications 14 à 17,
**caractérisé en ce que** l'étrier (211) est réalisé de façon déformable élastiquement.

19. Dispositif de préhension orthopédique selon la revendication 1 en combinaison avec la revendication 9, 10 ou 11,
**caractérisé en ce que** le cadre (11) est couplé au deuxième élément de doigt (321, 322).
